# EUROPEAN PATENT APPLICATION

(11) **EP 0 857 972 A1**
(43) Date of publication of application: **12.08.1998**
(21) Application number: 98200167.9
(22) Date of filing: 22.01.1998
(51) Int. Cl.: G01N 33/53, G01N 33/50, C07K 16/18

(54) **Immunoassay for the detection and quantitation of toxins causing paralytic shellfish poisoning**

(30) Priority: 24.01.1997 CL 14397
(71) Applicant: Tepual, S.A., Recoleta, Santiago (CL); Centro de Estudios Cientificos de Santiago, Santiago de Chile (CL)
(72) Inventor: Jaimovich, Enrique, Las Condes, Santiago (CL); Latorre, Ramon, Las Condes, Santiago (CL)
(74) Representative: Ungria Lopez, Javier

(57) **Abstract**

This invention relates to the development of an immunoassay for the detection and quantification of paralytic shellfish poisoning (PSP) toxins. The accumulation of paralytic toxins in mollusks is a seasonal phenomena that occurs with great variability. Since the ingestion of these toxins may cause death. a detection system to prevent the consumption of contaminated mollusks is desirable. At present, only one paralytic toxin, saxotoxin (STX) can be detected with available immunoassays. However, STX is not the principal toxin present in mollusks. and thus its detection and quantification does not account for the total toxicity of contaminated shellfish. With this invention we describe for first time an immunoassay able to detect all paralytic toxins. these include saxitoxin (STX), neosaxitoxin (NEO), gonyautoxins (GTXs), Cs and other derivatives. Using this immunoassay, total toxicity of all PSP-contaminated mollusks can be determined Additionally, the presence of toxins in dinoflagellates, protozoan, crustacean, shellfish, algae, bacteria, fish, or any other species or sample containing paralytic toxins, can be detected. In this application various immunoassays, methods for antibody production and uses of these immunoassays are described.

## Description

### TECHNICAL FIELD

This invention relates to the development of an immunoassay for the detection and quantification of paralytic shellfish poisoning (PSP) toxins.

### BRIEF DESCRIPTION OF THE STATE OF THE ART

The neurotoxin accumulation in bivalve mollusks from dinoflagellates can create a serious risk to human health. The ingestion of contaminated mollusks may cause the paralytic shellfish poisoning (PSP); a serious condition that may cause death (Kao 1966). Originally, it was thought that this phenomena was restricted to tropical waters, but in the last years it has become a worldwide problem (WHO, 1984). In the last 40 years there have been reported cases of PSP contamination in South Pacific, Central America, Southeast Asia, Europe, and Australia. This tendency suggests that the phenomena is increasing. Furthermore, PSP is not only restricted to bivalve mollusks but also to bivalve predators such as snails, Chilean abalons, crabs and others (Yasumoto *et al.*, 1981; Arakawa *et al*, 1995).

PSP is due to tetrahydropurine-like toxins. The first paralytic toxin was isolated from Alaska clams (*Saxidomas giganteus*) (Schantz *et al*., 1957) and was named saxitoxin (STX) (Schuett and Rapoport, 1962). Since then, all paralytic toxins have been known as saxitoxins. At present, *Saxidomas* is the only known species that selectively concentrates STX (Shimizu *et al*., 1978).

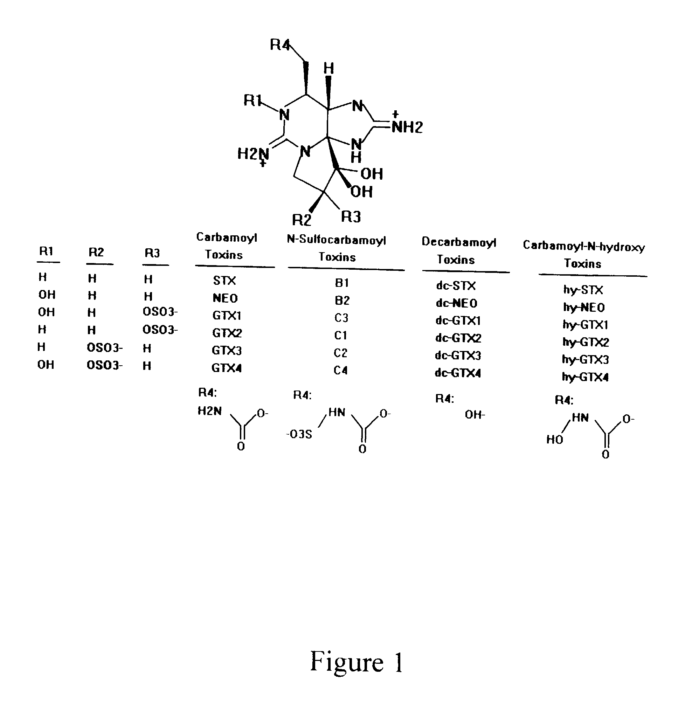

At least 18 types of PSP toxins have been described (Fig 1), primarily in marine dinoflagellates mollusks feeding on toxic algae, and the predators of these mollusks. Normally, a great variety of saxitoxins are found in a individual mollusk. However, carbamoyl (STX, NEO, GTX1, GTX2, GTX3 y GTX4) and N-sulfocarbamoyl (B1 (GTX5), B2(GTX6), C1, C2, C3 y C4) toxins are predominant (Hall and Reichardt, 1984; Shimizu and Hsu, 1981; Yasumoto, 1985). In some mollusks, decarbamoylated toxins (dcSTX, dcNEO, dcGTX2 y dcGTX3) and carbamoyl-N-hydroxylated toxins (hySTX and HyNEO) also contribute to the total toxicity (Sullivan *et al.*, 1983; Harada *et al*., 1983; Arakawa *et al.*, 1995). Among these toxins, decarbamoyl gonyautoxin-1 (dc-GTX1), dcGTX4, carbamoyl-N-hydroxy gonyautoxin-1 (hyGTX1), hyGTX2, hyGTX3 and hyGTX4, have been proposed but not isolated as paralytic toxins (Fig 1).

The absolute toxicity of these toxins varies considerably. Carbamoyl toxins and carbamoyl-N-hydroxy toxins are the most toxic, while N-sulfocarbamoyl toxins are less toxic (5 to 100 times less) (see table 1). Because a large number of closely related compounds are responsible for PSP, both the detection and the exact quantification of total toxicity in mollusks is complicated (Oshima *et al.,* 1992). However, interconversion of one into another form of toxin can be easily achieved. N-sulfocarbamoyl toxins can be converted to carbamoyl toxins by acid hydrolysis, or converted to decarbamoyl toxins enzymatically (Hall, 1982). Both of these conversions results in a substance substantially more potent. Enzymatic hydrolysis of carbamates to decarbamoyl toxins occurs at very low rate with a little change in potency (Hall, 1982). It is important to note that even when the total amount of toxin in a given sample remains constant, the concentration of individual toxins and therefore the total toxicity, will vary with time and can increase or decrease substantially.

**Table 1.**

| Specific Toxicity (mouse units/µmol) of paralytic toxins. | | |
|---|---|---|
| Toxin | Specific Toxicity (Mouse units/µmol) | Reference |
| STX | 2,480 | Oshima *et al.*, 1992 |
| NEO | 2,300 | Oshima *et al.,* 1992 |
| GTX1 | 2,470 | Oshima *et al.*, 1992 |
| GTX2 | 890 | Oshima *et al.*, 1992 |
| GTX3 | 1,580 | Oshima *et al.*, 1992 |
| GTX4 | 1,800 | Oshima *et al*. 1992 |
| B1 (GTX5) | 136 | Harada *et al.*, 1983 |
| B2 (GTX6) | 108 | Harada *et al.*, 1983 |
| C1 | 15 | Oshima *et al.*, 1992 |
| C2 | 240 | Oshima *et al.*, 1992 |
| dcSTX | 1,220 | Oshima *et al.,* 1992 |
| dcGTX2 | 380 | Oshima *et al.*, 1992 |
| dcGTX3 | 940 | Oshima *et al.*, 1992 |
| hySTX | 1,740 | Arakawa *et al.*, 1995 |
| hyNEO | 1,490 | Arakawa *et al.*, 1995 |

Although toxicity in bivalve mollusks is seasonal, (it follows the dinoflagellate growth pattern), toxicity in a specific location is extremely unpredictable. A specific geographic location can be toxin-free for years but, if hydrographic conditions trigger dinoflagellate proliferation, toxic mollusks can appear. The unpredictable nature of PSP forced the implementation of a wide and careful monitoring program of mollusk toxicity. These monitoring studies involve both collecting and periodical analysis of shellfish. Generally, when toxicity is above 80 µg STX equivalent/100 g of mollusk meat, the geographical location is closed to mollusk harvesting. Monitoring processes continue until toxicity drops, at which point harvesting can resume. All PSP monitoring programs require intensive sampling using a large number of samples. Thus, fast and exact protocols for analysis, allowing quick determination of toxicity levels would streamline this process..

Sommer and Meyer's (1937) pioneer studies on PSP allowed the development of a mouse bioassay, and established the basis for a standardized assay presently used all over the world (Helrich, 1990). This assay involves the extraction of the toxin by boiling shellfish in hydrochloric acid 0.1 N. One mL of this extract is then injected into standardized rats. The time between the injection and mouse death is used as toxicity measure. This assay has several problems, including the large number of mouse used, the interference of high salt contained in the samples, lack of sensitivity (the limit for detection is only 40 µg STX eq./100 g), and a variability around 20%. These problems have forced the search for alternative analytical techniques for the detection and quantification of PSP toxins, which could be useful in the shellfish monitoring programs.

Alternative techniques for PSP toxin detection are:
a) Binding assays to sodium channels
b) Chemical assays.
c) Column chromatography analysis.
d) Thin layer chromatography analysis.
e) Electrophoresis analysis.
f) HPLC analysis.
g) Immunoanalysis.

### a) Binding assay to sodium channels.

In this assay, pharmacological activity of saxitoxins is exploited. Toxins bound to sodium channels, preventing sodium influx and therefore membrane depolarization. Davio and Fontelo (1984) described an assay based on the competitive displacement of radioactive STX from rat brain membranes. This assay is highly sensitive (detection limit around 0.2 ppb STX) and is able to detect other sodium channel binding toxins, for example tetrodotoxin.

### b) Chemical assays.

These assays are based in the generation of colored or fluorescent toxin derivatives. Colorimetric methods, either based on the reaction with picric acid (McFarren *et al.,* 1958) or with 2,3-butanedione (Gershey *et al.,* 1977). In general, these methods are insensitive (100 µg STX eq./100 g), and require tedious purification steps with many interferences.

The fluorimetric method for PSP toxin detection is based on the ability of H₂O₂ or peryodate oxidation revert to its pirimidopurine derivatives (Bates and Rapoport, 1975; Jonas-Davies *et al.,* 1984). These methods are very sensitive (<1 µg STX eq./100 g) and specific for PSP toxins. These methods include (1) extraction, (2) cleaning by ion exchange, (3) oxidation of toxins, and (4) fluorescent measurements steps.

### c) Column chromatography analysis.

This technique was initially developed as a toxin purification procedure. A toxin net charge separation in an ionic exchange column is used. Weak cationic exchangers, such as GC-50™, IRC-50™, Bio-Rex 70™ are preferred. Using acetic acid gradients, toxins are partially separated, and eluted in the following order: B2, B1, GTX1/4, GTX2/3, NEO and STX (Shimizu *et al.*, 1975; Hall, 1982). Generally, the toxin profile is not completely resolved, but resolution is sufficient to allow the detection of the sample heterogeneity. Toxin detection in the column eluent is made using a mouse bioassay (Shimizu *et al.*, 1975; Boyer *et al.,* 1979) or by using the fluorescent method (Ikawa *et al.*, 1982). Polymeric resins such as Bio-Gel P^{™} and Sephadex G™ for toxin separation are also described (Shimizu *et al*., 1975; Buckley *et al.*, 1976; Hall, 1982), but toxins are eluted approximately in the reverse order to the ionic exchange columns, with C toxins eluting last.

### d) Thin layer chromatography analysis.

These methods use paper (Mold *et al.*, 1957) and silica (Proctor *et al*., 1975; Buckely *et al.,* 1976) chromatography procedures. In both cases toxin and interferents are resolved. The most common method uses silica gel plates and a mix of pyridine, ethyl acetate, water and acetic acid. Once separated by chromatography, plates are dried, sprayed with 1% (v/v) hydrogen peroxide, and then heated at 100°C for 30 min. Toxins are visualized by UV.

### e) Electrophoresis analysis.

Because toxins are charged molecules, they can be separated by electrophoresis. Usually, electrophoresis is used as a complementary tool to chromatography (Oshima *et al*., 1976; Boyer *et al.,* 1979; Ikawa *et al.*, 1985). Normally cellulose acetate plates and a great variety of buffers are used. Toxins are detected by the oxidation-fluorescense system. Recently a PSP electrophoretic analysis system has been developed (Thibault *et al.,* 1991), but it is only useful for partially purified toxins analysis. Furthermore, although it is quantitative and quick, sample concentration must be high (1 µg/mL) in order to detect the presence of toxins by UV at 200 nm. Thus, the total amount of toxins injected must be 20 pg. The migration order is: dcSTX, STX, NEO, 11-OH-STX, GTX2, GTX3, GTX1, and GTX4.

### f) HPLC analysis.

The HPLC method is based on an ion pairing chromatographic separation of toxins in a RP8 column. Afterwards, a post column derivatization by alkaline oxidation is carried out, and toxins are fluorimetrically, detected. (Sullivan *et al.,* 1988; Oshima *et al*., 1988). The most successful HPLC method is the one described by Oshima *et al.* (1988). In this method acid extracts of shellfish (the same used for mouse bioassay) are separated by chromatography in a Sep Pak C18™ column (Waters Co.) and then deproteinized by ultrafiltration (Millipore Ultrafree membrane C3GC™, exclusion 10,000 PM). The eluent is injected into an RP8 HPLC column, eluted at 0.8 mL/min with buffer A (2 mM sodium 1-heptanosulfonate in 10 mM ammonium phosphate pH 7.2), for GTXs and dcGTXs, and eluted with buffer B (A:acetonitrile = 9:1), for STX, NEO and dcSTX. Toxin detection is done by post column derivatization with 0.4 mL/min 7 mM periodic acid in 50 mM sodium phosphate pH 9.0, heating at 65°C in a 10 m Teflon™ tube (0.5 mm i.d.), and 0.4 mL/min of 0.4 M acetic acid, and then detected in a fluorescence detector (ex 330 nm; em 390 nm).

### g) Immunoanalysis.

The application of immunological techniques for saxitoxin detection was first described by Johnson *et al*. (1964) and Johnson and Mulberry (1966). In these studies, STX was coupled to bovine serum albumin (BSA) using formaldehyde and antibodies were raised in rabbits. With those anti-STX antibodies, a bentonite flocculation or hemaglutination assay was developed. This assay was very specific for STX but lacks of enough sensitivity for being a regular analysis system.

Carlson *et al*. (1984) developed a radioimmunoassay (RIA) able to detect low levels of STX. However, no cross reactivity was observed to NEO and other PSP toxins. This RIA detects only between 8 to 33% of sample total toxicity (measured by mouse bioassay). These discrepancies between RIA and mouse bioassay was expected, since the assayed mollusks came from east and west coast of North America having GTXs as predominant toxins, and not STX and NEO.

Chu and Fan (1985) reported the application of an immunoenzymatic assay (ELISA) for STX. This test has a sensitivity of 2 to 10 pg of STX. In this ELISA assay, NEO has a cross reactivity of 16%. However, GTX1 did not present cross reactivity, indicating that the sulfate sustituent present in GTXs and Cs toxins affects the cross reactivity. As it was observed in the RIA assay, this ELISA underestimate the toxicity values.

Renz and Terplan (1988) developed an indirect competitive immunoassay. The antibodies were obtained from immunized rabbits with STX-KLH (haemocyanine). A sensitivity of 20 pg/mL STX was observed. Antiserum presented only a 16% of cross reactivity with NEO. Usleber *et al.* (1991) developed a direct ELISA immunoassay. This assay, using the same antiserum of Renz and Terplan (1988) has been shown to have 10 times higher sensitivity. Cross reactivity with GTX1, GTX4 and B2 was not observed, while NEO exhibited a 10% cross reactivity.

Since shellfish toxicity is due to the combining effect of numerous different toxins. an immunoassay developed with anti-STX antibodies to measure shellfish "total toxicity", is not feasible. Low cross reactivity of antiserum with other paralytic toxins is a recurrent problem. This lack of cross reactivity had impeded the development of immunoassays that quantify the total toxicity present in shellfish.

The most promising STX immunoassay described thus far is one developed in Canada (Cembella *et al.*, 1990) which resulted in a commercially available system. This immunoassay is based on the inhibition of coupled-enzyme absorption. Briefly, the assay involves the competition between enzyme labeled-STX and sample PSP toxins for an immobilized anti-STX antibody, followed by the color development the conjugated enzyme. A decrease in color intensity compared with a negative control indicates the presence of PSP toxins. Serum cross reactivity correlates with the relative toxicity of various saxitoxins. However, although NEO bound at comparable level to GTX3, its binding is apparently reversible. Thus this assay may show an apparent drop of NEO concentration present in the sample, and thus there is the possibility of false-negative results.

The present invention is intended to overcome the problems inherent in prior art methods by an immunoassay-based system for both deection and quantification of paralytic toxins. Because each antibody is specific for each toxin, and because PSP is producied by a veriety of toxins, the use of a single antibody for both detection and quantification is not recommended. Thus, the present invention uses a combination of antibodies each of which recognizes the specific type of toxin. The combined detection of toxins using several antibodies allows the quanitifcation of total toxicity with a good correlation with mouse bioassay.

The invention as claimed provides an immunoassy for the detection and quantification of paralytic shellfish poisoning (PSP) toxins by which immunoassay the individual or combined forms STX, NEO, GTX1, GTX2, GTX3, GTX4, B1, B2, C1, C2, C3, C4 as well as the respective decarbamoyl and carbamoyl-N-hydroxyl derivatives is detected, and the total toxicity of the sample is established by using anti-STX, anti-GTX1-4, anti-Neo, anti C1-4, Anti B1/B2, anti-dcGTX1-4, antidcSTX, anti-dcNEO, anti-hySTX, and/or anti-hyGTX1-4 antibodies in combined or separate form.

The invention also provides a method for producing anti-PSP toxin antibodies by
(a) preparing an immunogen by coupling PSP toxin(s) with an immunologically active protein by using a chemical coupling system,
(b) inocculating said coupled PSP toxin(s) into an animal,
(c) obtaining antibodies from the animal,
(d) screening the specific anti-PSP toxin antbodies by means of titratio of the animal sera using an antigen correspondign to a conjugate different from that used as immunogen, the conugate being developed with a protein which is immunologically not related to that which is used as immunogen, and coupled by a chemical process which is preferredly different from that used in the elaboration of the immunogen.

Specific embodiments of the invention are disclosed in the detailed description of the prefered embodiments as well as in the dependent claims appended to the present description.

The invention will hereinafter be described by way of some preferred embodiments with reference to the drawings appended in which
fig. 1 shows structures of paralytic shellfish poisoning toxins;
fig. 2 shows indirect ELISA for sera titration and for cross reactivity determination and binding reaction to PSP-BSA conjugate;
fig. 3 shows competitive ELISA with PSP-peroxidase;;
fig. 4 shows anti-STX serum title determination using the third and eigth bleedings of STX-Concholepas concholepas haemocyanin (CCH) inocculated rabbits;
fig. 5 shows anti-GTX serum using the third and sixth bleedings of GTXs-CCH inocculated rabbits;
fig. 6 shows anti-C1/C2 serum title determination using the third and sixth bleedings of C1/C2-CCH inocculated rabbits,
fig. 7 shows anti-Neo serum title determination using the third and fifth bleedings of NEO-CCH inocculated rabbits.

### DETAILED DESCRIPTION OF THE PREFERRED EMBODIMENTS.

All previous work attempting to develop an immunodetection system for paralytic toxins has used anti-STX antibodies, while the detection of other non-STX toxins exploited the cross reactivity of other PSP toxins to anti-STX antibodies. However, all anti-STX antibodies obtained so far, except those descnbed by Cembella *et al*. (1990), have a very low cross reactivity with non-STX toxins. Since the shellfish toxicity is generally due to non-STX toxins, the use of these antibodies has been very limited. In this invention we avoid this difficulty by generating several antibodies, each of them against different PSP toxins. The combined use of these antibodies allows us to recognize and quantify all groups of toxins, thus determining the toxin profile and calculating with high precision the total toxicity of the sample.

The generation of this multiple antibody-based immunodetection system was done using the following procedure :

### 1.- Purification of toxins.

Purification of toxins was done using previously described standard procedures (Mold *et al.,* 1957; Schantz *et al*., 1957; Hall, 1982; Laycock *et al.*, 1994). The purified toxins were obtained using shellfish hepatopancreas, toxic dinoflagellates and bacteria. Organisms were selected for the purification of a particular type of toxin. For example, shellfish hepatopancreas were selected to purify GTXs toxins, and dinoflagellates for STX and NEO toxins.

Shellfish hepatopancreas (clams, mussels, giant mussels, pacific clams, oysters, scallops, sea asparagus, razor clams, etc.) (1 Kg) were homogenized in acetic acid 0.1 M (1 L), and heated to a temperature ranged between 80-100° C for 5 to 30 min. After cooling, precipitated proteins were separated by centrifugation. Supernatants were extracted 2 to 5 times with dichloromethane (500 mL each). Aquose phases were concentrated by rotatory evaporation to a 200 mL final volume, and then separated by chromatography on a column containing activated charcoal (500 g) and celite (500 g). The column was washed with 2 to 5 volumes of water, and sample was eluted with 20% (v/v) ethanol and 1% (v/v) acetic acid. One liter fractions were collected, and toxins were detected by mouse bioassay. Toxin containing fractions were pooled, concentrated by rotatory evaporation and lyophilized.

Toxic dinoflagellates (*Alexandrium* spp., *Gymnodinium* spp., *Pyrodinium* spp.) were grown in seawater-culture media using conditions similar to those described by Hall (1982). Cells were collected by filtration or centrifugation, and stored frozen at -20° C (20 to 30 g wet weight cells in 100 mL acetic acid 0.1M). Toxins were extracted by cell sonication; cell rupture was monitored using a microscope. Cellular debris were removed by centrifugation (10,000 to 20,000 x g by 20 to 60 min) and supernatants were reextracted with a similar volume of acetic acid 0.1M. Extracts were pooled and lyophilized.

Bacteria (*Vibrio* spp., *Bacillus* spp., *Moraxella*, spp., *Aeromonas* app., *Pseudomonas* spp., *Aphanizomenon flos-aquae*) were resuspended in acetic acid 0.1M (5 to 20 g in 100 mL), placed on ice, and sonicated 5 to 10 times at 1 min intervals. The homogenate was centrifuged 40,000 x g for several hours until all solid material was pelleted. Pellets were resonicated and recentrifuged as described above. The pooled extracts were concentrated by rotatory evaporation resulting in a final volume of up to 30-40 mL. One volume of 95% ethanol was added to the resulting solution and then centrifuged 20,000-40,000 x g for 30 to 120 min. Supernatant was concentrated and lyophilized.

Purification of toxins was done by column chromatography lyophilized residues were resuspended in acetic acid 0.1M and loaded to a Bio-Gel P-2™ (Bio Rad Laboratories) column preequilibrated with acetic acid 0.1M. Elutions were done with acetic acid 0.1M. Collected fractions were analyzed by HPLC using the procedure described by Oshima *et al.* (1988), pooled and separated in 3 fractions : Group A (STX and NEO), Group B (GTXs), and Group C (C1 and C2), and lyophilized.

Group A lyophilized residue was resuspended in 10 mL water and 0.2 mL of 2M Tris HCl pH 7.5 was added, and then separated by chromatography in a Bio-Rex-70™ (protonated form, Bio Rad Laboratories) preequilibrated in water. STX and NEO were eluted using a 0 to 3 M acetic acid linear gradient. Fractions were analyzed by HPLC and toxin containing fractions were lyophilized separately. Toxins were resuspended in 1-2 mL of water, passed through C-18 Sep Pak™ (Waters Co.) and reseparated by chromatography in the Bio-Rex-70™ column as described above. The purest fractions of this second chromatography were used as source of STX and NEO. This group of toxins and this procedure were also used to separate dcSTX and dcNEO toxins.

Group B lyophilized residues were resuspended in 10 mL of water and loaded onto a Bio-Rex-70™ column, prepared using the same procedure described above. GTXs were eluted with an isocratic gradient with acetic acid 0.05M. Fractions were analyzed by HPLC and pooled in group B1 (containing GTX1 and GTX4 toxins), group B2 (containing GTX2 and GTX3 toxins) and group B3 (containing *B*1 and *B*2 toxins). After lyophilization, toxins were resuspended in 1-2 mL of water, passed slowly through C-18 Sep Pak™ (Waters Co.) and reseparated by chromatography in the Bio-Rex-70™ column. This second separation was repeated as many times as necessary to remove the contaminant toxins in all groups. Pure fractions of GTX1/GTX4 in the group B1, GTX2/GTX3 in the group B2 and *B*1/*B*2 in the group B3, were pooled and used as source of mixed toxins GTX1/GTX4, GTX2/GTX3 and *B*1/*B*2. GTX1, GTX2. *B*1 toxins were separated from GTX4, GTX3 and *B*2 toxins, respectively, using a Carboxymethyl-Sephadex CM10™ column chromatography, and eluted with a 0 to 0.1M acetic acid linear gradient. In some cases it was possible to separate, by Bio-Rex-70™ chromatography, a fraction containing the GTXs decarbamoylated toxins, i.e dcGTXs.

Group 3 lyophilized residues were resuspended in 10 mL acetic acid 0.1M and separated by chromatography in a Bio-Gel P-2™ (Bio Rad Laboratories) column preequilibrated with acetic acid 0.1M. Elution was done with acetic acid 0.1M. Collected fractions were analyzed by HPLC and separated in pure C1 and C2 fractions as well as mixed C1/C2 fractions.

### 2.- Immunogen generation.

Immunogens were generated by coupling different toxins with a carrier protein. Bovine seroalbumin (BSA), ovoalbumin, haemocyanin (keyhole limphets haemocyanin [KLH] ; *Concholepas concholepas* haemocyanin [CCH]; *Limulus polyphemus* haemocyanin [LPH]; etc.), casein, lactalbumin, poly alanine-lysine (synthetic polypeptide), among others, were used as carrier proteins.

Immunogens were coupled using formaldehyde (Johnson *et al.*, 1964; Renz and Terplan, 1988), glutaraldehyde (Cembella, 1990), maleimidobenzoyl-N-hydroxysuccinimide ester (Puizillout and Delaase, 1981), and/or carbodiimide (Hoare and Koshland, 1967; Bauminger and Wilchek, 1980; Goodfriend *et al.*, 1964). The method selected allows the transformation of the toxins to toxoids without a significant change in structure.

Toxins used in coupling were :
A) Each of the purified paralytic toxin. For instance : STX, NEO, GTX1, GTX2, GTX3, GTX4, C1, C2, *B*1, *B*2, dcSTX, dcNEO, dcGTXs.
B) Mix of toxin groups. For instance, Group A (STX and NEO), Group B (GTX1, GTX2, GTX3, GTX4, *B*1 and *B*2), Group B1 (GTX1 and GTX4), Group B2 (GTX2 and GTX3), Group B3 (*B*1 and *B*2), Group C (C1 and C2), mix of Groups B1 and B2 (GTX1, GTX2, GTX3 and GTX4), and the decarbamoyl toxins dcSTX, dcNEO, dcGTX1, dcGTX2, dcGTX3 and dcGTX4.
C) Mix of several toxin groups, forming a toxin profile typically found in toxic shellfish, bacteria or dinoflagellates. For instance a mix of STX, NEO, GTX1, GTX2, GTX4, *B*1, *B*2, C1 and C2. In some cases decarbamoyl toxins were also included.

### 3.- Antibody generation.

Generation of antibodies against the paralytic toxins coupled to carrier proteins was done in rabbits (polyclonal antibodies) and BALB/c mouse (monoclonal antibodies). In both cases, complete Freund coadyuvant, incomplete Freund coadyuvant and aluminum hydroxide were used.

### 3.1.- Polyclonal antibodies.

Polyclonal antibodies were generated in rabbits. Preimmune bleeds were extracted and evaluated in all rabbits. First inoculation was done inoculating 0.1 to 1 mg of immunogen in complete Freund coadyuvant, subcutaneously. Subsequent inoculations were done by subcutaneous injections of 0.1 to 1 mg of immunogen in incomplete Freund coadyuvant or with aluminum hydroxide. 3 to 10 inoculations were done in total, every 7 to 15 days period. Antibody titers against the immunogen were evaluated after each inoculation except the first.. Blood samples were obtained by puncturing the rabbit ear, 7 - 14 days after each inoculation. Titers were determined by ELISA. In some cases it was necessary to do a last intravenous or intramuscular injection of the immunogen 0.1 to 2.0 mg without coadyuvant, 2 - 5 days or 10 - 14 days before the final bleeding, respectively.

Final bleeds were obtained by cardiac punction. Blood samples were coagulated at 37° C, by 15 min at 1 h, and then incubated at 4° C by 15 min at 1 h. Sera containing antibodies were obtained by centrifugation at 2,000 - 10,000 x g by to 20 min at 4° C, aliquotated and stored frozen at -20° C.

### 3.2.- Monoclonal antibodies.

Generation of monoclonal antibodies was done according to Norman *et al.*, (1985) and Köhler and Milstein (1975). Six to ten weeks old BALB/c mice were injected, via intraperitoneal, with 5 to 100 µg of immunogen in complete Freund coadyuvant. Afterwards, mice were reinjected 1 to 4 times, with 5 to 100 µg of immunogen in incomplete Freund coadyuvant, every 7 to 15 days. In some cases, 2 to 3 days prior to the fusion, it was necessary to boost the immunogen with an intravenous injection. Antibody secreting cells were isolated from spleen and mixed with myeloma cells. After cells were centrifuged, they were fused with polyethylene glycol. Fused cells were removed from the polyethylene glycol, diluted in selective culture media (HAT) and plated. After a week, supernatants of hybridoma containing plates were obtained and antibodies against paralytic toxins were detected.

Once plates that contain anti-paralytic toxin antibodies producer clones were identified, they were expanded and cloned according to the methodology described by Harlow and Lane (1988). The last expansions were obtained through the generation of an ascitic tumor, obtained by inoculating hybridomas of mouse peritoneum.

### 4.- Titration of antibodies.

Rabbit polyclonal antibody titers, and evaluation of both hibridoma culture media supernatants and ascitic liquids, were done using [³H]-STX displacement assay, or by ELISA and/or IRMA. The antigens used for ELISA and IRMA assays were prepared by coupling the toxins with a protein and method different to those used for immunogen preparation Thus, we can be assured that the antigen-antibody reactions take place only within the hapten-toxin part of coupled antigen and not the whole molecule. For example, if, haemocyanin or polyalanine-lysine were used as carriers and toxins were coupled with formaldehyde for immunogen generation, then antigen generation was carried out using BSA or casein as carriers and toxins, coupled with carbodiimide.

High affinity antibodies, specially high affinity monoclonal antibodies, were screened using the [³H]-STX displacement assay. As indicated in item 4.1.3., the assay description, only high affinity antibodies are able to displace STX from rat brain membrane sodium channels.

### 4.1.- Immunoassays.

### 4.1.1.- ELISA and IRMA

The ELISA and IRMA techniques developed for the paralytic toxins detection, can be classified in 2 groups, defined by whether the antigen or the antibody was bound to the solid phase.

If the antibody was bound to the solid phase, then the method is described an **antigen competitive assay.** For this assay a tracer is required, normally corresponding to both radioactive or enzyme (usually peroxidase or alcaline phosphatase) labeled toxins, pure or mixed. Antibodies are fixed to a solid matrix. Tracer competes with toxin from samples or standards, for the binding sites in the antibodies. Sample toxin displaces the tracer toxin from the antibodies. The larger the amount of toxin present in the sample, larger the displacement, generating an inverse correlation between the detected signal and the amount of toxin present in the sample. Using standard samples it is possible to calibrate the quantification of each toxin, either separately or grouped..

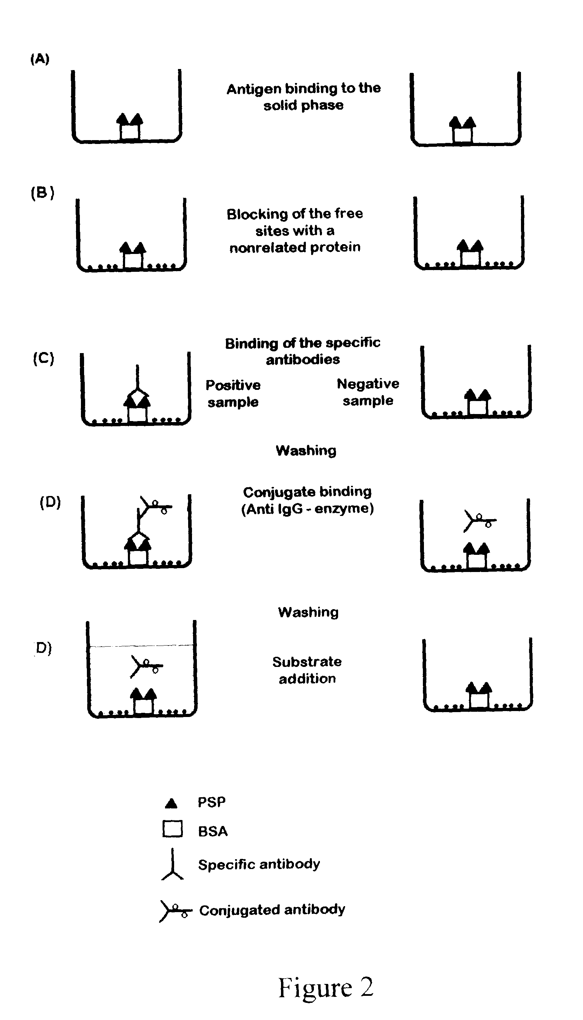

If the antigen was bound to the solid phase, then the method is described as an **antibody binding assay**. This assay is considered **direct** when the antibody used to recognize the antigen is itself labeled, either enzymatically or radioactivelly. This assay is considered **indirect** when the antibody used to recognize the antigen is detected using a second antibody (an anti-antibody) that is labeled, either enzymatically or radioactivelly. The indirect antibody binding assay is a classic procedure for antibody detection and titration (see figure 2). This procedure involves the binding of the protein coupled toxin to a solid phase, and then this toxin-protein complex or conjugate is allowed to react with the antibody. Both the protein and coupling method used in the conjugate must be different to those used in the generation of immunogen during immunizations to assure that the antibody recognizes only the hapten-toxin part of the antigen. The antibody bound to the toxin-protein complex is detected using a second antibody, corresponding to an anti-antibody labeled with an enzyme (ELISA) or radionucleotide (IRMA).

A variation of the antibody binding assay can be used to measure the presence of toxin in a sample. This assay is called an **antibody binding competitive inhibition assay**. The toxin, pure or mixed, and previously coupled to a carrier protein is bound to a solid phase. The protein used in the coupled toxin and the coupling method must be different to those used in the generation of the immunogen during immunizations. As indicate above, this will insure that the antigen-antibody interaction will occur only by the hapten-toxin moiety of the molecule. For the inhibition assay, first antibodies and samples are mixed and incubated. Here, the antibody-toxin bindings are favored. Next, these antibodies are mixed with antigens coupled to a solid phase. After washing, the antibodies bound to the solid phase are detected using a second antibody, ie. an anti-antibody enzymatically or radioactivelly labeled. This is an **indirect competitive assay**. A **direct competitive assay** uses the same procedure describe above, but uses an antibody possessing the enzymatic or radioactive label, thereby eliminating the need for a secondary antibody. The assay is calibrated with standard toxins, and generates an inverse relation between the toxin present in the sample and the detected signal.

Assays correspond to ELISA if the label used for detection corresponds to an enzyme, and to IRMA, if the label corresponds to a radioactive label.

### 4.1.2.- RIA and EIA.

RIA and EIA immunoassays used to both detect and quantify paralytic toxins corresponding to competence assays between a labeled (radioactivelly or enzymatically) and a non labeled toxin from a sample or standard. The reaction is very similar to those occurring in the antigen competitive ELISA, but in this case the antigen-antibody reaction is done in solution. Once the equilibrium is reached, the antigen-antibody complex is separated from the tracer precipitating the immune complex with polyethylene glycol, activated charcoal, etc. or by capture using an anti-antibody bound to a solid phase, for instance Sepharose™ or carboxymethyl cellulose. The detected signal is inversely proportional to the amount of toxin present in the sample. If the label is radioactive, then the assay is a RIA, and if the label is an enzyme then the assay is an EIA.

### 4.1.3.- [³H]-STX displacement assay.

Displacement assays exploit the ability of anti-PSP toxins antibodies to displace bound labeled toxin, usually radioactive, from sodium channels present in rat brain membrane preparations. This assay is very useful for detecting the presence of antibodies with high affinities for toxins. An antibody can displace toxins from the sodium channel, only if the affinity of the antibody to the toxins is equal or higher than that of the toxin for the sodium channel.

This assay involves the simultaneous incubation of the antibodies with [³H]-STX and a rat membrane preparation (containing sodium channels). After equilibrium is reached, an aliquot of the solution is filtered through a glass fiber filter, such as GF/F™ (Whatman), and washed rapidly. The radioactivity of each filter, determined by a liquid scintillation counter, corresponds to the amount of [³H]-STX bound to rat brain membranes. The larger the amount of radioactivity present in the filter, the smaller the capacity of antibody to displace toxins.

### 4.1.4.- Inhibition of the agglutination.

This assay is based on the capacity of PSP toxins to inhibit the agglutination of latex particles or other material, when they are incubated with a predetermined quantity of antibodies against paralytic toxins. The agglutination of these toxin-covered substances particles is produced by cross linking these substances to anti-PSP toxin antibodies. Inhibition is produced through a competition between the particle bound toxins and substance toxins. Using sample dilutions, and calibrating these dilutions with standards of known concentrations, it is possible to determine the concentration of toxins in the sample.

### 4.1.5.- Inhibition of the immunoprecipitation.

This assay is based on the capacity of PSP toxins to inhibit the precipitation of protein coupled toxins, when incubated with a predetermined quantity of antibodies against paralytic toxins. The precipitation of protein coupled toxins is produced by cross linking these proteins in a similar way to that described for agglutination. Inhibition is produced through a competition for the antibody between the protein coupled toxin and the toxin from the sample. Using sample dilution, and calibrating these dilutions with a standard of known concentration, it is possible to determine the concentration of toxins of the sample. The sample dilution can be made in liquid phase or in solid phase through immunodiffusion or electrodiffusion assays.

### 4.2.- Coupling of toxins to a marker enzyme or radionuclide.

### 4.2.1.- Coupling of toxins to a marker enzyme.

Paralytic toxins can be coupled to a marker enzyme through a chemical reaction involving the amino or hydroxyl structural groups, with any functional group on the marker enzyme. In general, the amino groups are more utilized for this purpose because they are easier targets for chemical modification. The marker enzymes can be peroxidase, phosphatase, glucose oxidase, β-galactosidase and β-glucuronidase, among others.

Coupling reagents are usually cross linking reagents. These reagents can couple an amino group on the toxin with an amino group on the protein (amino-amino), (homobifunctional reagents); or an amino group on the toxin with another functional group on the protein, for example sulfhydryl lamino-sulfhydryl), carboxyl (amino-carboxyl), hydroxyl (amino-hydroxyl), amide (amino-amide), guanidinium (amino-guanidinium), (heterobifunctional reagents). The amino-amino homobifunctional reagents used are glutaraldehyde derivatives; imidoester homobifunctional cross linkers, such as dimethyladipimidate, dimethylpimelimidate, dimethylsuberimidate, dimethyl-3,3'-dithiobis propionimidate, among others; or well N-hydroxysuccinimidyl ester cross linker derivatives, such as ethylene glycobis (succinimidylsuccinate), ethylene glycobis (sulfosuccinimidylsuccinate), disuccinimidyl tartrate, disulfosuccinimidyl tartrate, disuccinimidyl glutarate, disuccinimidyl suberate, bis (sulfosuccinimidyl) suberate, dithiobis (succinimidyl propionate), dithiobis (sulfosuccinimidyl propionate), bis [2-succinimidyloxycarbonyloxy) ethyl] sulfone, bis [2-(sulfosuccinimidooxycarbonyloxy) ethyl] sulfone, among others.

Within heterofunctional reagents containing amino-sulfhydryl reagents are: succinimidyl 4-(N-maleimidomethyl) cyclohexane-1-carboxylate, sulfo-succinimidyl 4-(N-maleimidomethyl) cyclohexane-1-carboxylate, *m*-maleimidobenzoyl-N-hydroxysuccinimide ester, *m*-maleimidobenzoyl-N-hydroxysulfo succinimide ester, succinimidyl 4- (*p*-maleimidophenyl)-butyrate, sulfosuccinidyl 4-(*p*-maleimidophenyl) butyrate, bismaleimidohexane, N-(γ-maleimidobutyryloxy) succinimide ester, among others. Within those are amino-carboxyl reagents are different carbodiimide derivatives. Within those that are amino-amide and amino-guanidinium reagents are the formaldehyde and its derivatives.

### 4.2.2.- Radioactive labeling of paralytic toxins.

Paralytic toxins, mainly saxitoxin and neosaxitoxin, can be easily marked with tritium. Labeling is carried out by an interchange of hydrogens at position 11 of STX or NEO with tritium using tritiated water, following by HPLC purification. The [11-³H] saxitoxin can be also obtained commercially at Amersham (Little Chalfont, Buckinghamshire HP7 9NA, England).

### 4.3.- Determination of antibody specificity.

As indicated above, sera titer determinations against the respective toxins were carried out mainly through an antibody binding assay. To determine if antibodies specifically recognize the toxin-hapten fraction of the coupled protein used in this assay, an antibody binding competitive inhibition assay was done. In this inhibition assay, the protein coupled toxin was bound to the solid phase and washed with a solution containing the respective antibodies plus a predetermined and increasing quantity of the same assayed toxin in free and soluble form. Free toxin and protein coupled toxin compete for the same antibody binding site. Increasing the concentration of free toxin in solution inhibits the antibody binding to the solid phase. This binding inhibition indicates the existence of a specific recognition of the antibody for the analyzed toxin.

### 4.4.- Determination of antibody cross reactivity.

Antibody cross reactivity was determined using the immunoassays previously described (see item 4.1), in general, binding inhibition assays or direct binding assays. In the binding inhibition assay, a labeled toxin (radioactivelly or enzymatically) competes with several paralytic toxins (generally in solution), for the binding sites of toxin-specific antibodies, generally bound to a solid phase. Cross reactivity appears as a reduction of antibody binding to the solid phase, indicating an inhibition of the ability of antibodies to the recognize the corresponding toxin. . Results are expressed as a percentage of cross reactivity compared with a control. In the indirect binding assays, each antibody is reacted with several PSP toxins coupled with proteins bound to a solid phase. Antibody binding is detected using a second antibody (an anti-antibody) radioactivelly or enzymatically labeled. In this case the degree of antibody binding is directly proportional to the cross reactivity that presents the antibody with other toxins (see figure 2).

### 5.- Development of an immunoassay for both toxin detection and quantification in samples of dinoflagellates, bacteria, shellfish and other marine products.

### 5.1.- Description of samples.

Samples suitable for analysis using this immunoassay include dinoflagellates, protozoan, crustaceans, shellfish, algae, bacteria, fish or any another material, liquid or live being that contains paralytic toxins.

### 5.2.- Preparation of samples.

Sample extracts can be prepared using the same procedure as that for mouse bioassay. Briefly, samples are homogenized and mixed with one volume of HCI 0.1N. After verifying that pH is <4.0, samples are boiled for 5 min, cooled, and pHed again (pH 4.0). The volume lost by evaporation is recovered by adding HCl 0.1N. Samples are centrifuged and supernatants are utilized for both detection and quantification of toxins. Sample neutralization is achieved through dilution of at least 1: 100 in an appropriate buffer, for example PBS.

### 5.3.- Preparation of ELISA, IRMA, RIA, EIA, agglutination inhibition and immunoprecipitation inhibition systems, for the total toxicity detection of PSP contaminated samples.

Once the sample is prepared, the paralytic toxin presence and quantity can be detected using one of the systems described at item 4.1.

Within the proposed methods, those corresponding to the solid phase assays (ELISA and IRMA) and agglutination and/or immunoprecipitation inhibition assays are the most appropriate for use on a large scale. The aqueous phase assays (RIA and VIA) require an immune complex separation step, that is sometimes troublesome.

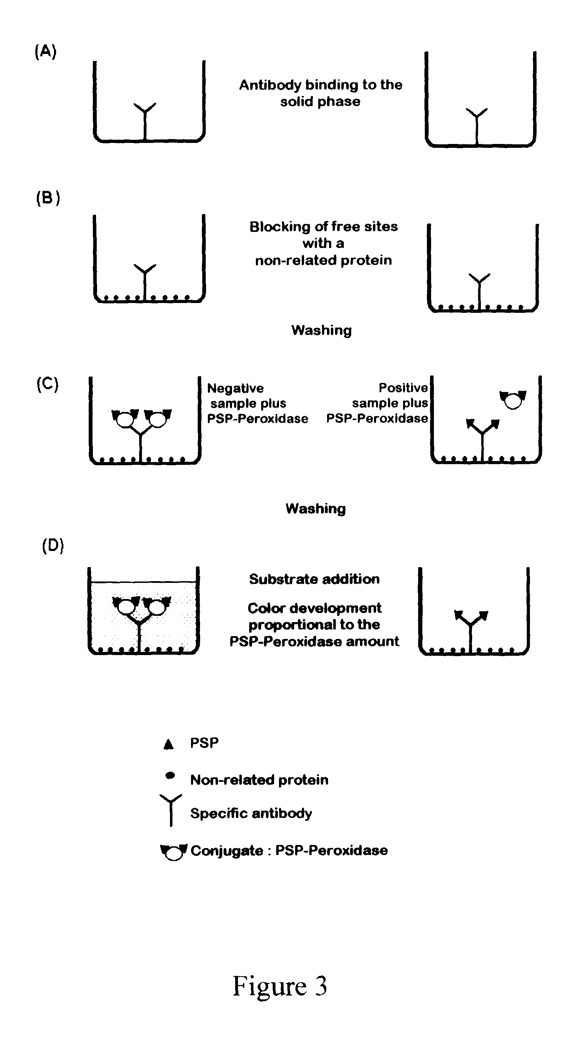

Appropriate solid phase assays are the **competitive inhibition of the antibody binding assay** (the antigen bound to the plate) and the **antigen competitive assay** (the antibody bound to the plate). The use of an enzymatic detection system is s easier, safer and less expensive than the corresponding radioactive assays. The figure 3 details an antigen competitive ELISA assay, in which the labeled enzyme is peroxidase.

Both assays, the competitive inhibition of the antibody binding assay and the antigen competitive assay, can be evaluated in two ways :

### 5.3.1.- Individual detection of toxins and determination of the total toxicity of the samples by determining the individual contribution of each type of toxin.

This method consists of the creation of a calibration curve for each group or specific type of toxin. The samples are prepared as described at the item 5.1 and the toxins are analyzed individually. Each calibration curve is used to quantify the amount, in µmoles, of each group or specific type of PSP-toxin at the sample, allowing the complete toxin profile of the sample to be determined. In other words, this allows the determination of which toxins are present and in what quantity. The total toxicity of the sample is calculated through the sum of the individual toxic contributions for each group or type of toxin. Using the table 1 of the descriptive memory, it is possible to determine the comparative toxicity of each toxin, expressed in mouse units per µmol of toxin. Finally, similarly to the mouse bioassay, these mouse units can be used to calculate the final toxicity expressed as µg STX equivalents/ 100 g of shellfish (Helrich, 1990).

### 5.3.2.- Total detection of toxins and total toxicity determination of the sample using an average toxicity factor.

This method consists of mixing the different types of antibodies and forming an unique antisera useful to recognize all toxins. The relative proportions of antibodies used will approximately correspond to the same proportion of those toxins found in the contaminated shellfish. In Chile, this proportion is approximately STX 10-20%; NEO 5-10%; GTXs 75-80%; C1-C4 0-10%. Other known toxins, contribute less than 5%, and therefore, do not contribute substantially to the total toxicity. For the analysis of other samples, (for example of other origins or profiles), a different mixture of antibodies will be necessary. All antibody mixes function in the same manner as a serum with cross reactivity to all the PSP toxins.

These antibody mixes can also be manufactured, as previously indicated, using proteins coupled with mixtures of toxins instead of pure toxins for the inoculations. In fact, the inoculation of a proteins coupled with a mixture of toxins, in a similar proportion to that found at the nature), allowed us to generate antibodies with the same characteristics as those obtained with a mixture of individual sera. This method of antibody preparation allows the efficient control, of "cross reactivity" presented by a serum against the different PSP-toxins.

Preparation of the detection systems can be done in the same way as that described in item 5.2.1., with some modifications. In the case of antigen competitive assay, the antigen used to compete for the antibodies binding sites, also correspond to mixtures of enzyme-labeled toxins. These mixtures are made in the same proportions as those of the antibodies. In the antibody binding competitive inhibition assay, the antigen bound to the plate is also a mixture of protein-coupled toxins.

In both cases, the calculation of the total toxicity of the sample is carried out through a factor that is empirically determined by several crossed immunoassay and mouse bioassay calibrations.

### EXAMPLE 1. Elaboration of polyclonal antibodies against paralytic toxins.

### 1.- Preparation of the toxins.

Toxins STX, NEO, GTXs (63% GTX2, 31% GTX3, 4% GTX1, and 2% GTX4) and C1/C2 (98% C2, 2% C1) were obtained from mussels contaminated with red tide, collected at the XII Region - Chile. The mussels were washed with abundant water, shucked and the hepatopancreas removed. Two Kg of hepatopancreas were homogenized in 2 L acetic acid 0.1 M with an ultraturrax, and boiled for 10 min. After cooling, the samples were centrifuged, and the supernatant was extracted 4 time with 1 L of dichloromethane each. The aqueous phase was concentrated by rotatory evaporation to 400 mL final and was separated by chromatography in a column containing a mixture of 1 Kg activated charcoal and 1 Kg celite. The column was washed with 4 L of water, and eluted with 10 L of a ethanol 20% (v/v) and acetic acid 1% (v/v) solution. 10 fractions of 1 L were collected and the toxin presence was detected by mouse bioassay. The fractions were pooled, concentrated by rotatory evaporation and lyophilized. The toxins were purified by Bio-Gel P2™ and Bio-Rex-70^{™} columns chromatography, according to the procedures described in the descriptive memory.

### 2.- Coupling of the PSP toxins to the immunogenic carriers.

Toxin binding to the immunogenic carriers proteins was carried out using formaldehyde as coupling reagent. The formaldehyde destroys the toxic activity, without changing the chemical structure of the toxins. The toxins were coupled to *Concholepas concholepas* haemocyanin (CCH). 0.25 µmoles of toxin were resuspended in 250 µL of sodium acetate 0.1N pH 4.2, and subsequently 1 mg of CCH and 20 µl of formaldehyde were added. The whole mixture was stirred for 72 h at 4°C was dialyzed for 3 days against distilled water at 4°C to eliminate the formaldehyde and the non-bounded low molecular weight fractions.

### 3.- Coupling of the PSP toxins to BSA.

Preparation of the toxin-BSA conjugate (PSP-BSA) bound to the solid phase in the ELISA assays was carried out in the same manner. However, in this case bovine seroalbumin (BSA) was utilized. BSA was solubilized in 1 mL of sodium acetate 0.1M pH 4.2, and after adding 0.25 µmoles of toxin and 50 µL of formaldehyde (37%), the solution was agitated during 3 days at room temperature. The conjugated was dialyzed during 3 days at 4°C in acetic acid 1 mM.

### 4.- Immunization of the rabbits and collection of sera.

Rabbits were immunized using the following protocol:
- Day 0: Primary immunization, with 75 µg of toxin-CCH in complete Freund coadyuvant via intradermal.
- Day 10: Secondary immunization, with 32.5 µg of coupling in incomplete Freund coadyuvant via intradermal
- Day 22: Secondary bleeding. Sera collection.
- Day 24: Tertiary immunization, with 32.5 µg of coupling in incomplete Freund coadyuvant via intradermal.

According to this protocol, the sera were obtained 12 days after each immunization and each inoculations occurred 14 days after each immunization. The toxin immunized rabbits were inoculated and bled according to the immunization protocol up to a total of 9 times, via intramuscular in physiologic serum. After 12 days the animals were bled to white.

### 5.- Indirect ELISA binding assay for the sera titration.

ELISA plates were activated over night at 4°C with PSP-BSA (10 µg/mL). After saturating the plates with PBS-fetal bovine serum 3% (v/v) for 1 h at room temperature, they were washed 4 times with PBS-Tween 20 0.05% (v/v). Both immune and preimmune sera of each animal were diluted in PBS-BSA 0.01% (v/v) and incubated for 2 h at room temperature. In order to eliminate the excess of not bound antibodies, the plates were washed again using the same procedure. The determination of the antigen-antibody reaction was carried out adding a second antibody (goat anti-rabbit IgG conjugated to alkaline phosphatase) and incubating for 90 min to room temperature. The reaction was developed using as substrate p-nitrophenylphosphate and incubating during 30 minutes at room temperature. The absorbance was detected at 405 nm.

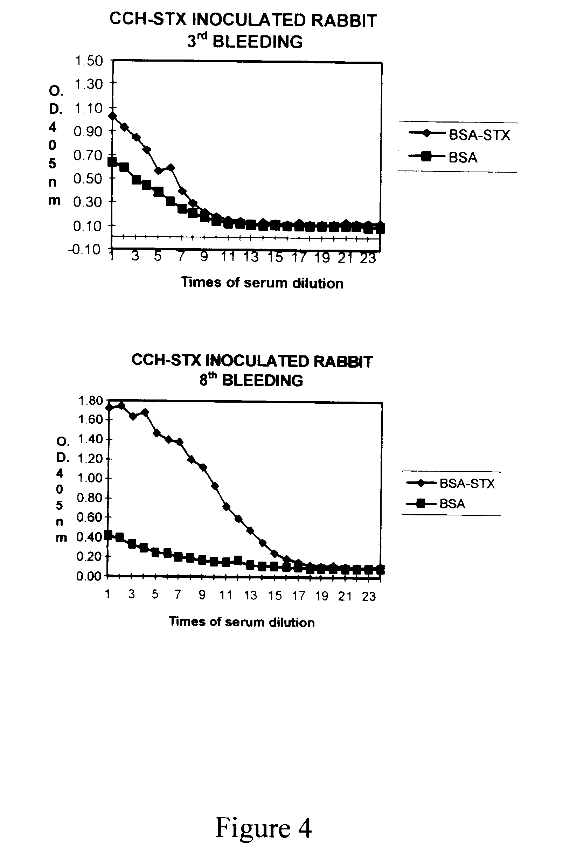

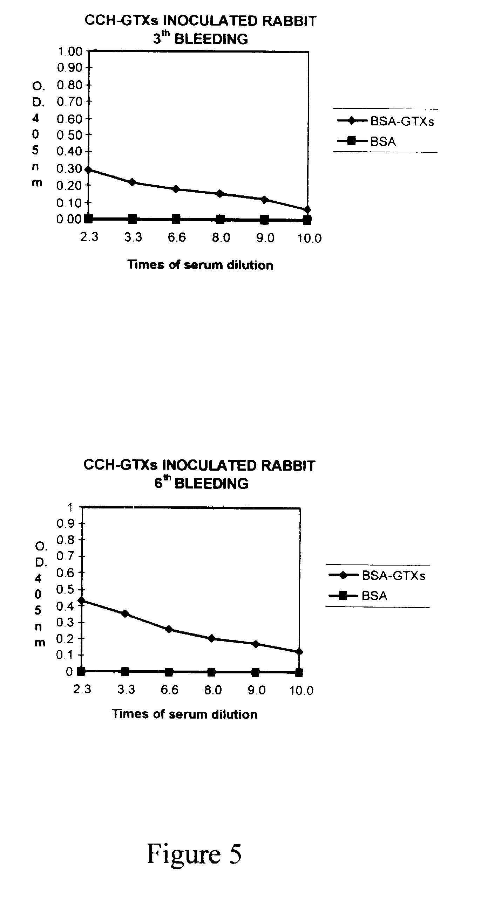

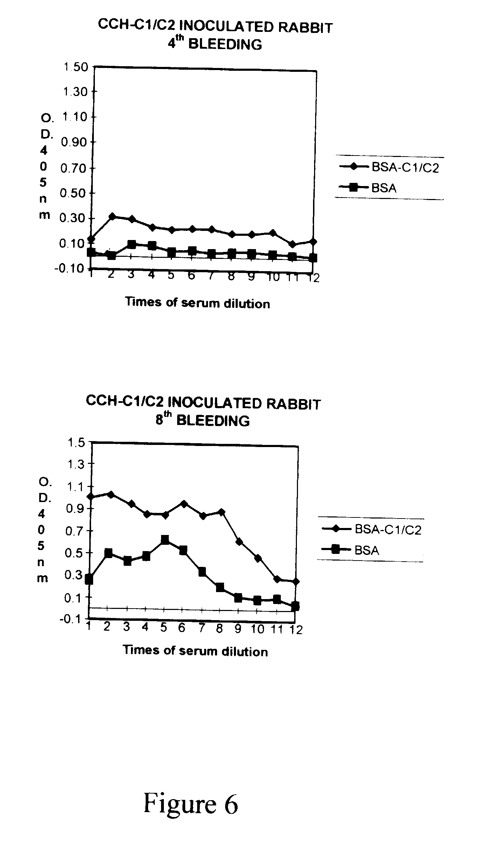

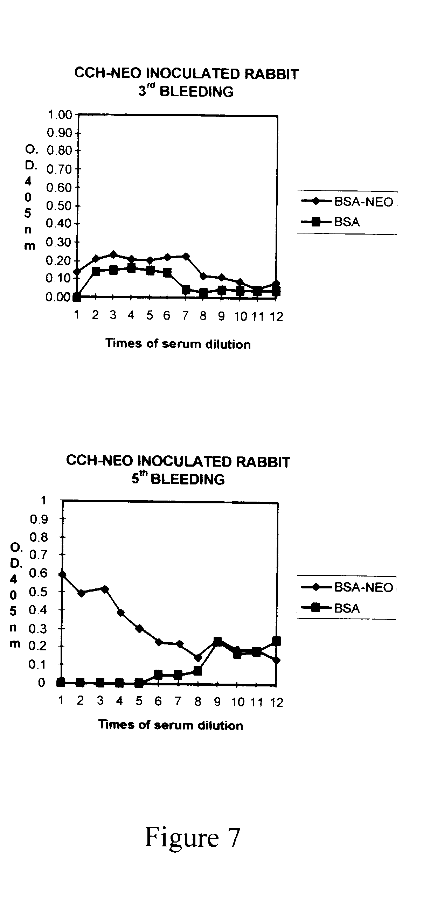

Using this procedure, we determined the titer of every serum against each respective toxins. For instance, figures 4, 5, 6 and 7 show the curves of the anti-STX, anti-GTXs, anti-C1/C2 and anti-NEO antibody titles, respectively.

Graphs show that while the animals were immunized, the antibody sera concentration increased. These antibodies were shown to be specific against the respective toxins as they poorly recognize the carrier protein (BSA) in comparison to the coupled toxins (PSP-BSA).

### 6.- Indirect ELISA binding assay for determining the sera specificity.

Antibody specificity was determined by inhibition assays using similar procedures as those described in the sera title determination assays. However, in this case, the sera were preincubated in solutions containing known PSP concentrations to block their binding to the PSP-BSA adsorbed to the ELISA plate. The antibody binding inhibitions were detected as a decrease of the O.D. during the color development step. The tables 2, 3, 4 and 5 show the results of the anti-PSP sera specificity determinations.

**Table 2.**

| Antibody binding inhibition ELISA assay for anti-STX serum specificity determination. | | |
|---|---|---|
| Utilized immune serum | O.D. 405 nm* BSA-STX antigen | O.D. 405 nm Control BSA |
| Immune Serum Dil 1/1000 | 1.53 | 0.00 |
| **+ STX (500 ng/mL)** | **0.01** | **0.00** |

| | | |
|---|---|---|
| * each result is the average of 3 determinations. | | |

Table 2 shows clearly that, in the STX-CCH immunized animals, antibodies able to specifically recognize STX were produced. This inhibition reaction was specific, since the presence of STX in solution was able to inhibit the reaction and prevented the color development. This anti-sera has a high titer of specific antibodies which allows use in higher dilutions than 1/1000.

**Table 3.**

| Antibody binding inhibition ELISA assay for anti-GTXs serum specificity determination. | | |
|---|---|---|
| Utilized immune sera | O.D. 405 nm* BSA-GTXs antigen | O.D. 405 nm Control BSA |
| Immune Serum Dilution 1/250 | 0.300 | 0.045 |
| + GTXs (500 ng/mL) | **0.014** | **0.035** |
| Immune Serum Dilution 1/500 | 0.201 | 0.036 |
| + GTXS (500 ng/mL) | **0.004** | **0.038** |
| Immune Serum Dilution 1/1000 | 0.095 | 0.045 |
| + GTXs (500 ng/mL) | **0.006** | **0.036** |

| | | |
|---|---|---|
| * each result is the average of 3 determinations | | |

Table 3 shows that the immunization with GTXs-CCH induced the production of specific antibodies, since the presence of GTXs in solution is able to displace the reaction and inhibit the color development. The antibody title reached in this case was lower that for the STX.

**Table 4.**

| Antibody binding inhibition ELISA assay for anti-C1/C2 serum specificity determination. | | |
|---|---|---|
| Utilized immune sera | O.D. 405 nm* BSA-C1/C2 antigen | O.D. 405 nm Control BSA |
| Immune Serum Dil 1/250 | 0.458 | 0.105 |
| + C1/C2 (500 ng/mL) | **0.090** | **0.089** |
| Immune Serum Dil 1/500 | 0.308 | 0.120 |
| + C1/C2 (500 ng/mL) | **0.098** | **0.100** |
| Immune Serum Dil 1/1000 | 0.250 | 0.080 |
| + C1/C2 (500 ng/mL) | **0.076** | **0.070** |

| | | |
|---|---|---|
| * each result is the average of 3 determinations | | |

Table 4 shows that the immunization with C1/C2-CCH induced the production of specific antibodies in the immunized animals, because the presence of C1/C2 in solution was able to inhibit the reaction and prevent color development. The antibody titer was quite good, but inferior to that of STX.

**Table 5.**

| Antibody binding inhibition ELISA assay for anti-NEO serum specificity determination. | | |
|---|---|---|
| Utilized immune sera | O.D. 405 nm* BSA-NEO antigen | O.D. 405 nm Control BSA |
| Immune Sera Dil 1/250 | 0.288 | 0.090 |
| + NEO (500 ng/mL) | **0.061** | **0.095** |
| Immune Sera Dil 1/500 | 0.190 | 0.070 |
| + NEO (500 ng/mL) | **0.057** | **0.060** |
| Immune Sera Dil 1/1000 | 0.095 | 0.050 |
| + NEO (500 ng/mL) | **0.050** | **0.055** |

| | | |
|---|---|---|
| * each result is the average of 3 determinations | | |

Table 5 shows that the immunization with NEO-CCH induced the production of specific antibodies in the immunized animals, because the presence of NEO in solution was able to inhibit the reaction and prevent the color development. The antibody title was quite good, but inferior to that of STX.

### 7.- Determination of the anti GTXs and anti NEO antibodies cross reactivity.

The cross reactivity of these antibodies was tested in an indirect binding ELISA assay, using the procedure described in 5).

The anti GTXs sera have cross reactivity to C1/C2-BSA, and a very low cross reactivity against NEO-BSA. The anti NEO sera have cross reactivity against STX-BSA, and a low cross reactivity against C1/C2-BSA and GTXs-BSA.

The cross reactivities might be explained by the structural similarity between GTXs and C1/C2, and between NEO and STX. The GTXs toxins and the C1/C2 toxins only differ in the sulfate of the carbamoyl group. This group is distant from the central core of the toxin, which is exactly the same for both types of toxins. STX and NEO only differ in a hydroxyl group.

### 8.- Determination of the anti-STX antibodies cross reactivity.

An antigen competitive ELISA assay was used for the determination of the anti-STX antibody cross reactivity. The corresponding antibodies were bound to the plates, saturated with fetal bovine serum, washed, and incubated with known concentrations of GTXs, C1/C2 or NEO together with the STX-peroxidase conjugate. The percentage of binding inhibition corresponded to the observed percentage of cross reactivity. Table 6 shows the results of two sera anti-STX, obtained from different animals.

In general, the anti-STX sera have high cross reactivity against NEO. However for the other toxins (GTXs, C1/C2), this cross reactivity is very low. These results confirm that it is not possible to utilize only anti-STX sera to determine the shellfish total toxicity, since the low cross reactivity with other toxins would underestimate the total toxicity.

**Table 6.**

| Cross reactivity determination of two anti-STX sera against the toxins- PSP. | | |
|---|---|---|
| **TOXIN** | **Serum 1 anti- STX %*** | **Serum 2 anti-STX %** |
| STX | 100 | 95 |
| NEO | 37 | 19 |
| C1 | 0.4 | 0.2 |
| C2 | 0.3 | 0.05 |
| GTX2-3 | 8.4 | 0 |

| | | |
|---|---|---|
| * Each value corresponds to the average of 2 determinations. The results are expressed as percentage of reaction. | | |

### EXAMPLE 2. Elaboration of a paralytic toxin detection system by a immunocompetence assay.

This example describes an ELISA immunoassay based in a competitive system of direct antigen. In this assay the antibody binds to the solid phase, and the toxin competes for the binding sites with a peroxidase labeled toxin.

### 1.- Preparation of the conjugated Peroxidase- PSP.

0.27 µmol of PSP were dissolved in 250 mL of acetic acid 0.1 M and the mix was adjusted to pH 7.5 with carbonate/bicarbonate buffer pH 9.4. Subsequently 1 mg (0.023 µmol) of pre-activated peroxidase was added (Wilson *et al.,* 1978) and dissolved in 100 mL of distilled water. In general, a 1:11 (peroxidase:toxin) molar relationship gave the best results and generated assays with good sensibilities. 10 mL of reduction solution was added to the mixture and agitated for 1 h to room temperature. Subsequently, 20 µL of stop solution was added and mixed for another 15 min. The conjugated proteins were dialysed 1 day at 4°C against PBS and stored at -20°C in 50% (v/v) glycerol.

### 2.- Purification of the antibodies.

The anti-PSP antibodies were partially purified with an ammonia sulfate 0-50% fractionation. 10 mL of serum was diluted with 10 mL of PBS. While gently stirring at 4°C, solid ammonia sulfate was added to 50% saturation and the resulting suspension was stirred for 1 h at 4°C. Subsequently the suspension was centrifuged at 20,000 x g for 30 min at 4°C, and the pellet was dissolved in 20 mL of PBS. This solution was subjected to a new 0-50% ammonia sulfate fractionation, using the same procedure described above. The final pellet was dissolved in 5 mL of PBS and dialyzed over night against PBS at 4°C.

### 3.- Activation of the plates with the first antibody or second antibody.

PIERCE™ plates were activated adding anti-PSP sera, previously diluted with carbonate/ bicarbonate buffer pH 9.6, and incubated over night at 4°C. Subsequently, the plates were saturated with PBS-fetal bovine serum 3% (v/v) 1 h at room temperature and washed 4 times in PBS-Tween 20 0.05% (v/v).

In order to increase antibody binding to the plates, purified anti-PSP antibodies were used, according to the previously described protocol, or a protocol described by Usleber (1991). In the Usleber method, the plates were activated with an anti-rabbit IgG for 2 h at room temperature, and then saturated with PBS-FBS 3% (v/v) for 1 h at room temperature. The plates were washed 4 times with PBS-Tween 20 0.05% (v/v) and then dilutions of immune or preimmune sera were added and incubated over night at 4°C.

Once washed, plates were incubated with 50 µL of conjugated peroxidase-PSP (0.1 µg/mL) (control 100% of binding), in presence of known concentrations of PSP (standard curve, normally between 10-810 pg/mL), and in presence of 50 µL of samples that contain PSP (samples). In order to eliminate the excess of non bound conjugated, plates were washed as previously described and the reaction was revealed with tetramethyl benzidine in presence of H₂O₂. The absorbance was determined at 450 nm.

In the table 7 is shown the calibration curve for STX and GTXs. Similar calibration curves were obtained for NEO and C1/C2.

**Table 7.**

| Calibration curve for STX and GTXs using a competition ELISA assay. | | |
|---|---|---|
| Toxin concentration (pg/mL) | % O.D. STX calibration curve* | % O.D. GTXs calibration curve |
| 0 | 100.0 | 100.0 |
| 10 | 90.1 | 87.0 |
| 30 | 64.9 | 66.2 |
| 90 | 38.1 | 46.6 |
| 270 | 16.3 | 30.0 |
| 810 | 7.9 | 24.3 |

| | | |
|---|---|---|
| * Each value corresponds to the average of 3 determinations. | | |

Utilizing the previously described assay and with aid of the calibration curve it was possible to determine the presence of the different toxins in red tide contaminated shellfish samples and to determine their total toxicity.

The samples were prepared according to the same procedure described for the mouse bioassay (Helrich, 1990). All samples were analysed using mouse bioassay and an aliquot of this preparation was utilized for the immunoassay. The samples were diluted with PBS-BSA 1% according to the amount of toxin present in the sample, and corresponding to the dilution necessary to interpolate the O.D. into the respective calibration curves.

Table 8 shows the results of toxicity determinations of several samples. These samples were diverse red tide contaminated shellfish, and included mussels, oysters and clams. The individual amount of each toxin (STX, GTXs, NEO and C1/C2) was determined. The contribution of each toxin to the final toxicity final was calculated using the table 1 of the Descriptive Memory, where the relative toxicity of each toxin was described. The final result was expressed in µg equivalent of STX/ 100 g of shellfish.

**Table 8.**

| Determination of the total toxicity of red tide contaminated shellfish using an immunoassay and their comparison with mouse bioassay. | | | | | | | |
|---|---|---|---|---|---|---|---|
| Sample | Shellfish | Toxicity determined by immunoassay expressed as µg STX eq./100 g | | | | Total toxicity (STX+GTXs+NEO +C1/C2) | Toxicity determined by mouse bioassay |
| | | STX | GTXs | NEO | C1/C2 | (µg STX eq./100g) | (µg STXeq./100 g) |
| 1 | Oyster | 57 | 3 | 0 | 17 | 77 | nd* |
| 2 | Oyster | 58 | 805 | 32 | 10 | 905 | 879 |
| 3 | Clam | 22 | 720 | 20 | 10 | 772 | 607 |
| 4 | Mussel | 307 | 9,890 | 690 | 77 | 10,964 | 8,949 |
| 5 | Mussel | 380 | 21,070 | 649 | 268 | 22,367 | 15,674 |
| 6 | Clam | 109 | 5,838 | 360 | 123 | 6,430 | 5,623 |
| 7 | Mussel | 44 | 2,132 | 102 | 44 | 2,322 | 1,432 |
| 8 | Mussel | 20 | 912 | 56 | 18 | 1,006 | 1,125 |
| 9 | Mussel | 32 | 786 | 49 | 11 | 878 | 523 |
| 10 | Oyster | 6 | 1 | 0 | 3 | 10 | nd |
| 11 | Mussel | 12 | 1 | 0 | 10 | 23 | nd |

| | | | | | | | |
|---|---|---|---|---|---|---|---|
| *nd : not detected. | | | | | | | |

In general, the total toxicities detected by the immunoassay correlates very well with the toxicity obtained by the mouse bioassays. However, the values obtained by the immunoassays are always higher that those detected by the bioassays. This could be because in this example, the antibodies used have certain degree of cross reactivity with structurally similar toxins. If this were the case, the toxicity value, obtained by the addition of individual contribution of toxicity, overestimates the existent amount.

This system demonstrates the first immunoassay that is totally effective determining the profile of toxins present in red tide contaminated shellfish, and is useful for determining the total toxicity within a sample. Before this patent such results could only be achieved by HPLC analysis.

### REFERENCES

Arakawa, O., Noguchi, T. and Onoue, Y. 1995. Paralytic shellfish toxin profiles of xanthid crabs *Zosimus aeneus* and *Atergatis floridus* collected on reefs of Ishigaki island. Fisheries Science 61: 659-662.

Bates, H.A. and Rapoport, H. 1975. A chemical assay for saxitoxin the paralytic shellfish poison. J. Agric. Food Chem. 23:237-239.

Bauminger, S. and Wilchek, M. 1980. The use of carboiimides in the preparation of immunizing conjugates. Methods Enzymol. 70: 151-159.

Boyer, G.L., Fix-Wichmann, C.. Mosser, J., Schantz, E.J. and Schnoes, H.K. 1985. Toxins isolated from Bay of Fundy scallops. En "Toxic Dinoflagellate Blooms" (Taylor, D.L. y Seliger, H.H. Eds.) Elsevier/North-Holland, Amsterdam, pp. 373-376.

Buckley, L.J, Ikawa, M. and Sasner, J.J. 1976. Isolation of *Gonyaulax tamarensis* toxins from soft shell clams (*Mya arenaria)* and a thin-layer chromatographic-fluorometric method for their detection. J. Agric. Food Chem. 24: 107-111.

Carlson, R.E., Lever, M.L., Lee, B.W. and Guire, P.E. 1984. Development of immunoassays for paralytic shellfish poisoning: A radioimmunoassay for saxitoxin. En "Seafood Toxins" (Ragelis, E.P. Ed.) American Chemical Society, Washington D.C., pp 181-192.

Cembella, A.D., Parent, Y., Jones, D. and Lamourex, G. 1990. Specificity and cross-reactivity of an absorption-inhibition enzyme-linked immunoassay for the detection of paralytic shellfish toxins. En "Toxic Marine Phytoplankton" (Graneli, E., Anderson, D.M., Edler, L. y Sundstrom, B.G. Eds.) Elsevier Science Publishing Co., Inc., New York, pp. 339-344.

Chu, F.S. and Fan, T.S.L. 1985. Indirect enzyme-linked immunosorbent assay for saxitoxin in shellfish. J. Assoc. Offic. Anal. Chem. 68: 13-16.

Davio, S.R. and Fontelo, P.A. 1984. A competitive displacement assay to detect saxitoxin and tetrodotoxin. Anal. Biochem. 141: 199-204.

Gershey, R.M., Neve, R.A., Musgrave, D.L. and Reichardt, P.B. 1977. A colorimetric method for determination of saxitoxin. J. Fish. Res. Board Canada 34: 559-563.

Green, N., Alexander, H., Olson, A., Shinnick, T.M., Sutcliffe, J.G. and Lerner, R.A. 1982. Immunogenic structure of influenza virus hemagglutinin. Cell 28: 477-487.

Goodfriend, T.L., Levine, L. and Fasman, C. 1964. Antibodies to bradykinin and angiotensin: A use of carboiimides in immunology. Science 144: 1344-1346.

Hall, S. 1982. Toxins and Toxicity of *Protogonyaulax* from the northeast Pacific. Ph. D. Thesis. University of Alaska.

Hall, S. and Reichardt, P.B. 1984. Cryptic paralytic shellfish toxins. En "Seafood toxins" (Ragelis, E.P. Ed.). American Chemical Society, Washington, D.C. pp. 113-123.

Harada, T., Oshima, Y. and Yasumoto, T. 1983. Natural occurrence of decarbamoil saxitoxin in tropical dinoflagellates and bivalves. Agric. Biol. Chem. 47: 191-193.

Harlow, E., and Lane, D. (Eds) 1988. En "Antibodies. A Laboratory Manual". Cold Spring Harbor.

Helrich, K. 1990. "Official Methods of Analysis of the Association of Official Analytical Chemists", 15th ed. Arlington, VA: AOAC Inc., pp. 881-882.

Hoare, D.G. and Koshland, D.E. 1967. A method for the quantitative modification and estimation of carboxylic acid groups in proteins. J. Biol. Chem. 242: 2447-2453.

Ikawa, M., Auger, K., Mosley, S.P., Sasner, J.J., Noguchi, T. and Hashimoto, K. 1985. Toxin profiles of the blue-green alga *Aphanizomenon flos-aquae*. En "Toxic dinoflagellates" (Anderson, D., White, A,W. y Baden, D.G. Eds.) Elsevier, New York, pp. 299-304.

Johnson, H.M. and Mulberry, G. 1966. Paralytic shellfish poison: Serological assay by passive hemagglutination and bentonite flocculations. Nature 211: 747-748.

Johnson, H.M., Frey, P.A., Angelotti, R., Campbell, J.E. and Lewis, K.H. 1964. Haptenic properties of paralytic shellfish poison conjugated to proteins by formaldehyde treatment (29599). Proc. Soc. Exp. Biol. Med. 117: 425-430.

Jonas-Davies, J., Sullivan, J.J., Kentala, L.L., Liston, J., Iwaoka, W.T. and Wu, L. 1984. Semiautomated method for the analysis of PSP toxins in shellfish. J. Food Sci. 49: 1506-1509.

Kao, C.Y. 1966. Tetrodotoxin, saxitoxin and their significance in the studs of excitation phenomena. Pharm. Rev. 18: 997-1049.

Kitagawa, T. and Aikawa, T. 1976. Enzyme coupled immunoassay of insulin using a novel coupling reagent. J. Biochem, 79: 233-236.

Kohler G., and Milstein, C. 1975. Continuous cultures of fused cells secreting antibody of predefined specificity. Nature 256: 495-497.

Laycock, M.V., Thibault, P., Ayer, S.W. and Walter, J.A. 1994. Isolation and purification procedures for the preparation of paralytic shellfish poisoning toxin standards. Natural Toxins 2: 175-183.

Liu, F.T., Zinnecker, M., Hamaoka, T. and Katz, D.H. 1979. New procedures for preparation and isolation of conjugates of proteins and a synthetic copolymer of D-amino acids and immunochemical characterization of such conjugates. Biochemistry 18: 690-697.

McFarren, E.F., Schantz, E.J., Campbell, J.E. and Lewis, K.H. 1958. Chemical determination of paralytic shellfish poison in clams. J. Assoc. Offic. Anal. Chem. 41: 168-177.

Mold, J.D., Bowden, J.P., Stanger, D.W., Maurer, J.E., Lynch, J.M., Wyler, R.S., Schantz, E.J. and Riegal, B. 1957. Paralytic shellfish poison. VII. Evidence for the purity of the poison isolated from toxic clams and mussels. J. Am. Chem. Soc. 79: 5235-5238.

Norman, R.J., Poulton, T. and Chard, T. 1985. Monoclonal antibodies to human gonadotropin: Implications for antigenic mapping, immunoradiometrics assays and clinical applications. J. Clin. Endocrinol. and Metabolism 61: 1031-1037.

Oshima, Y., Fallo, W.E., Shimizu, Y., Noguchi, T. and Hashimoto, Y. 1976. Toxins of the *Gonyaulax* sp. and infested bivalves in Owase Bay. Bull. Jpn. Soc. Sci. Fish. 42: 851-856.

Oshima, Y., Sugino, K. and Yasumoto, T. 1988. Latest advances in HPLC analysis of paralytic shellfish toxins. En "Mycotoxins and Phycotoxins '88" (Natori, S., Hashimoto, K. y Ueno, Y. Eds.) Amsterdam, Elsevier Science Publishers B.V., pp. 319-326.

Oshima, Y., Bolch, C.J. and Hallegreaff, G.M. 1992. Toxin composition of resting cysts of *Alexandrium tamarense* (Dinophyceae). Toxicon 30: 1539-1544.

Proctor, N.H., Chan, S.L. and Trevor, A.J. 1975. Production of saxitoxin by cultures of *Gonyaulax catenella*. Toxicon 13: 1-9.

Puizillout, J.J. and Delaase, M.A. 1981. Radioimmunoassay of 5-hydroxyindole acetic acid using a iodinated derivative. J. Pharmacol. Exp. Ther. 217: 791-797.

Renz, V.V. and Terplan, G. 1988. Ein enzymimmunologischer Nachweis von Saxitoxin. Archiv für Lebensmittelhygiene 39: 25-56.

Schantz, E.J., Mold, J.D., Stranger, D.W., Shavel, J., Riel, F., Bowden, J.P., Lynch, J.M., Wyler, R.S., Riegel, B. and Sommer, H. 1957. Paralytic Shellfish poison. VI. A procedure for the isolation and purification of the poison from toxic clam and mussel tissues. J. Am. Chem. Soc. 79: 5230-5235.

Schuett, W. and Rapopport, H. 1962. Saxitoxin, the paralytic shellfish poison. Degradation to a pyrrolopyrimide. J. Am. Chem. Soc. 84: 2266-2267.

Shimizu, Y. and Hsu, C.P. 1981. Confirmation of the structures of gonyautoxins I-IV: correlation with saxitoxin. J. Chem. Soc. Chem. Comm. 7:314-315.

Shimizu, Y., Alam, M., Oshima, Y. and Fallon, W.E. 1975 Presence of four toxins in red tide infested clams and cultures *Gonyaulax tamarensis* cells. Biochem. Biophys. Res. Commun. 66: 731-737.

Shimizu, Y., Fallon, W.E., Wekell, J.C., Gerber, D. Jr. and Gauglitz, E.J. Jr. 1978. Analysis of toxic mussels (*Mytilus* sp.) from the Alaskan Inside Passage. J. Agric. Food Chem. 26: 878-881.

Sommer, H. and Meyer, K.F. 1937. Paralytic shellfish poisoning. Arch. Path. 24. 569-598.

Sullivan, J.J., Iwaoka, W.T. and Liston, J. 1983. Enzymic transformations of PSP toxins in the little neck clam (*Protothaca staminea*). Biochem. Biophys. Res. Comm. 114: 465-472.

Sullivan, J.J., Wenkell, M. and Hall, S. 1988. Detection of paralytic shellfish toxins. En "Handbook of Natural Toxins 3: Marine Toxins and Venoms" (Tu, A.T. Ed.) Marcel Dekker Inc., New York, pp. 87-106.

Thibault, P., Pleasance, S. and Laycock, M.V. 1991. Analysis of paralytic shellfish poisons by capillary electrophoresis. J. Chromatog. 542: 483-501.

Usleber, E. 1991. Direct enzymo-linked immunosorbent assays for the detection of the 8-ketotrichothecene mycotoxins deoxynivalonol, 3-acetyldooxynivalenol, and 15-acetyldooxynivalenol in buffer solutions. J. Agric. Food Chrom. 39: 2091

Usleber, E., Schneider, E. and Terplan, G. 1991. Direct enzyme immunoassay in microtitration plate and test strip format for the detection of saxitoxin in shellfish. Lett. Appl. Microbiol. 13: 275-277.

WHO. 1984. Aquatic (Marine and Freshwater) Biotoxins. Environmental Health Criteria 37. International Programme on Chemical Safety, World Health Organization, Geneva.

Wilson, B. and Nakane, P.K. 1978. Recent development in the periodate method of conjugating HPR to antibodies. In immunofluorescence and related attaining techniques ed Knapp. W Amsterdam: Elsevier.

Yasumoto, T. 1985. Recent progress in the chemistry of dinoflagellate toxins. En "Toxic dinoflagellates" (Anderson, D., White. A,W. y Baden, D.G. Eds.) Elsevier, New York, pp. 259-270.

Yasumoto, T., Oshima, Y. and Konta, T. 1981. Analysis of paralytic shellfish toxins in xanthid crabs in Okinawa. Bull. Japan. Soc. Sci. Fish. 47: 957-959.

## Claims

1. An immunoassay for the detection and quantification of paralytic shellfish poisoning toxins, that is able to detect, in both individual or combined forms STX, NEO, GTX1, GTX2, GTX3, GTX4, B1, B2, C1, C2, C3, C4 and their respective decarbamoyl and carbamoyl-N-hydroxyl derivatives, and the subsequent determination of the total toxicity of the sample,comprised of the utilization in combined or separated form of anti-STX, anti-GTX1-4, anti-NEO, anti-C1-4, anti-B1/B2, anti-dcGTX1-4, anti-dcSTX, anti-dcNEO, anti-hySTX, anti- hyNEO, and anti-hyGTX1-4 antibodies.

2. An immunoassay for the detection and quantification of paralytic shellfish poisoning toxins, according to claim 1, wherein said immunoassay could be carried out in solid phase, or in liquid phase.

3. An immunoassay for the detection and quantification of paralytic shellfish poisoning toxins, according to claim 2, wherein the immunoassay in liquid phase, could be a radioimmunoassay (RIA) or a enzyme immunoassay (EIA).

4. An immunoassay for the detection and quantification of paralytic shellfish poisoning toxins, according to claim 2, wherein the immunoassay in solid phase, could be an ELISA, an IRMA, an inhibition of agglutination assay or an inhibition of immunoprecipitation assay.

5. An immunoassay for the detection and quantification of paralytic shellfish poisoning toxins, according to claim 4, wherein the ELISA or the IRMA corresponds to a competitive antibody binding inhibition assay, that is, with the antigen bound to the plate, or to a antigen competitive assay, that is, with the antibody bound to the plate.

6. An immunoassay for the detection and quantification of paralytic shellfish poisoning toxins, according to claim 4 or 5, wherein the competitive antibody binding inhibition assay can be direct, when the used antibody to recognize the complex or conjugated toxin-protein is himself radioactive or enzymatically labeled, or can be indirect, when the used antibody to recognize the complex or conjugated toxin-protein is detected using a second antibody (an anti-antibody) that is radioactive or enzymatically labeled.

7. An immunoassay for the detection and quantification of paralytic shellfish poisoning toxins, according to claim 1, wherein the immunoassays can be carried out: through the determination of each one of the individual toxins and calculation the total toxicity through the addition of the toxicity contributions of each one of the toxins; or through the use of a mixture of the antibodies, as an non specific serum, for determining the total toxicity through the reaction of the complete mixture of toxins with the mixture of antibodies.

8. An immunoassay for the detection and quantification of paralytic shellfish poisoning toxins, according to claim 7, wherein the immunoassays for determining separately each one of the toxins is achieved through the use of their respective specific antibodies.

9. An immunoassay for the detection and quantification of paralytic shellfish poisoning toxins, according to claim 7, wherein the preferred mixture of antibodies is that that resemble the proportion of the toxins necessary to analyze.

10. An immunoassay for the detection and quantification of paralytic shellfish poisoning toxins, according to claim 7 or 9, wherein the mixture of antibodies could be achieved: mixing poly or monoclonal antibodies; or through the inoculation of a conjugated coupled with a mixture of toxins, in order to get a polyclonal serum that possesses the desired mixture of anti-toxin antibodies.

11. An immunoassay for the detection and quantification of paralytic shellfish poisoning toxins, according to claim 3 or 5, wherein both the EIA and the antigen competitive ELISA immunoassays, utilize a toxin coupled to an enzyme that can be alkaline phosphatase, peroxidase, glucose oxidase, β-galactosidase, β-glucuronidase, among others.

12. An immunoassay for the detection and quantification of paralytic shellfish poisoning toxins, according to claim 11, wherein the toxin coupled to an enzyme for the EIA and antigen competitive ELISA, can be whatever selected between STX, NEO, GTX1, GTX2, GTX3, GTX4, B1, B2, C1, C2, C3, C4 and their respective decarbamoyl or carbamoyl-N-hydroxyl derivatives, in individual form or in mixture using whatever of the possible combinations between these toxins

13. An immunoassay for the detection and quantification of paralytic shellfish poisoning toxins, according to claim 12, wherein the mixture of favored toxins is equal to the mixture of antibodies used in the immunoassay or equal to the mixture of toxins that is wanted to detect.

14. A method for the production of anti-PSP toxins antibodies, that involve the following steps:
a.- Preparation of an immunogen through the coupling of the PSP toxin(s) with an immunologically active protein, using a chemical coupling system
b.- Inoculation of the said coupled into an animal.
c.- Obtaining of the antibodies.
d.- Screening of specific anti-PSP toxin antibodies through the titration of the animal sera using an antigen that correspond a conjugate different to the used as immunogen, developed with a protein immunologically not related to the used as immunogen, and coupled by a chemical procedure, ideally, different to the used in the immunogen elaboration.

15. A method for the production of anti-PSP toxin antibodies, according to claim 14, wherein the preparation of the immunogen can be carried out using the pure toxins (STX, NEO, GTX1, GTX2, GTX3, GTX4, B1, B2, C1, C2, C3, C4 and their respective carbamoyl and carbamoyl-N-hydroxyl derivative) or mixtures of them.

16. A method for the production of anti-PSP toxin antibodies, according to claim 14, wherein the antibodies can be monoclonal or polyclonal antibodies.

17. A method for the production of anti-PSP toxin antibodies, according to claim 14 or 15, wherein it is possible to obtain specific sera for each type of toxins, through the inoculation of a conjugate of each one of the pure toxins, or to obtain sera that recognize simultaneously multiple toxins through: a) the sera mixture that have poly o monoclonal antibodies specific to each type of toxin; or b) through the inoculation of a conjugate coupled with a mixture of toxins.

18. A method for the production of anti-PSP toxin antibodies, according to claim 14 or 17, wherein regulating the presence or absence of toxins, and their amount in the conjugated immunogen, it is possible to regulate the specificity and cross reactivity of the produced polyclonal sera using said immunogen.

19. Uses of an immunoassay for paralytic toxin determination useful for both toxin profile and/or for samples total toxicity determinations.

20. Uses of immunoassay for paralytic toxin determination, according to claim 19, wherein the samples correspond to shellfish, crustaceans, algae, protozoa, bacteria, fish, dinoflagellate, or any another material, fluid or live being that contains paralytic toxins.
